# EUROPÄISCHE PATENTSCHRIFT

(11) **EP 0 927 203 B1**
(45) Veröffentlichungstag und Bekanntmachung des Hinweises auf die Patenterteilung: **13.06.2001**
(21) Anmeldenummer: 97909234.3
(22) Anmeldetag: 19.09.1997
(51) Int. Cl.: C08F 8/00, C07C 29/16, C07C 45/50

(54) **VERFAHREN ZUR HYDROFORMYLIERUNG**
HYDROFORMYLATION PROCESS
PROCEDE D'HYDROFORMYLATION

(30) Priorität: 20.09.1996 DE 19638796
(43) Veröffentlichungstag der Anmeldung: 07.07.1999
(73) Patentinhaber: BASF AKTIENGESELLSCHAFT, 67056 Ludwigshafen (DE)
(72) Erfinder: BLANKERTZ, Heinrich-Josef, D-67147 Forst (DE); GRENACHER, Armin, Volker, D-67112 Mutterstadt (DE); SAUER, Friedrich, D-67271 Obersülzen (DE); SCHWAHN, Harald, D-69168 Wiesloch (DE); SCHÖNMANN, Willi, D-67117 Limburgerhof (DE); ROEPER, Michael, D-67157 Wachenheim (DE)
(86) Internationale Anmeldenummer: EP9704907
(87) Internationale Veröffentlichungsnummer: WO9812235

(56) Entgegenhaltungen:
- EP-A- 0 046 564
- EP-A- 0 391 650
- WO-A-90/05711
- DE-A- 3 014 044
- DE-A- 4 404 742
- FR-A- 2 187 884
- GB-A- 2 055 371
- US-A- 2 815 390
- US-A- 3 993 615

## Beschreibung

Die Erfindung betrifft ein Verfahren zur Hydroformylierung von Olefinen von 12 bis 100 C-Atomen bei Drücken von 100 bis 400 bar und bei einer Temperatur von 100 bis 200°C mit einem Kobaltkatalysator und Abtrennung des Katalysators, wobei man die Extraktion des Katalysators in Gegenwart eines Emulsionsspalters mit einer nachgeschalteten Koaleszenzstufe durchführt.

Die Hydroformylierung von Olefinen unter Verwendung eines Kobaltkatalysators bei Drücken von 100 bis 400 bar und Temperaturen von 100 bis 200°C ist ein in der Technik wohlerprobtes Verfahren (Ullmanns Enzyklopädie der Technischen Chemie, Band 7, Seite 120ff) .

Dabei wird der Kobaltkatalysator in der Regel in Form des Acetates oder Formiates oder auch in Form löslicher Salze höherer Carbonsäuren z.B. als Kobaltethylhexanoat eingesetzt. Bevorzugt wird Kobaltformiat- oder Kobaltacetat über die wäßrigen Lösungen in Mengen von 0,1 bis 3 Gewichtsprozent bezogen auf das zu hydroformylierende Olefin zugeführt. Näheres ist der Monographie J. Falbe, New Syntheses with Carbon Monoxide, Springer Verlag, 1970, 162-165 zu entnehmen.

Aus Gründen der Wirtschaftlichkeit und der Befreiung des Hydroformylierungsprodukts vom Katalysator, muß das Kobalt, das in Form von Kobaltcarbonyl oder Kobaltcarbonylwasserstoff vorliegt, möglichst vollständig abgetrennt und in die Synthesestufe zurückgeführt werden.

Dazu wird üblicherweise gemäß den Angaben der DE-A 24 04 855 das Oxoreaktionsgemisch mit molekularem Sauerstoff in Gegenwart von wäßriger Säure behandelt. Dabei wird das Kobalt von der Oxidationsstufe -1 nach +2 oxidiert und kann sodann durch Extraktion mit der wäßrigen Lösung entfernt werden. Die Abtrennung des wäßrigen Extrakts erfolgt z.B. durch Dekantieren, in einem Phasentrenngefäß oder in anderen dafür geeigneten Einrichtungen.

Andere Verfahren (s. Ullmanns Enzyklopädie der Technischen Chemie, Bd. 7, S. 123) bedienen sich der thermischen Zersetzung der Kobaltcarbonyle zur Abtrennung aus dem Reaktionsgemisch. Man senkt dazu den CO-Partialdruck durch Entspannen des Reaktionsproduktes ab und leitet überhitzten Wasserdampf ein. Das abgeschiedene Kobalthydroxid, -oxid oder -metall wird dann mechanisch oder durch Auflösen in HNO₃ abgetrennt.

In einer anderen Ausführungsform wird der den Kobaltcarbonylwasserstoff enthaltende Reaktionsaustrag mit einer wäßrigen Natriumcarbonatlösung gewaschen, die den aciden Carbonylwasserstoff aufnimmt. Aus der wäßrigen Phase kann der Katalysator mit z.B. Schwefelsäure wieder in Freiheit gesetzt und rezirkuliert werden.

Es ist auch möglich verschiedene Entkobaltungsverfahren zu kombinieren. So wird gemäß DE-A 30 26 900 vorgeschlagen, in einem ersten Schritt den Kobaltcarbonylwasserstoff mit einer wäßrigen Kobalt-II-Salzlösung in Form des komplexen Salzes Co(Co(CO)₄)₂ aus der organischen Phase zu extrahieren und in einem zweiten Schritt Reste des Kobaltkatalysators durch Behandeln mit Luft und Säure zu entfernen.

Der vom Kobalt befreite Reaktionsaustrag kann dann in üblicher Weise weiter verarbeitet werden, z.B. durch Destillation, Hydrierung oder hydrierende Aminierung.

Während kurzkettige Olefine inzwischen überwiegend bei niedrigeren Drücken in Gegenwart von Rhodiumkatalysatoren hydroformyliert werden, hat die Hydroformylierung von höheren Olefinen mit 12 und mehr Kohlenwasserstoffatomen mit Kobaltkatalysatoren ihre Bedeutung behalten.

Eine Anwendung der Hydroformylierungsreaktion, unter Verwendung eines Kobaltkatalysators, die von besonderem Interesse ist, stellt gemäß EP-A 244 616 die Hydroformylierung von Polybutenen oder Polyisobutenen zu Polybutyl- bzw. Polyisobutylaldehyden, -alkoholen oder -estern dar.

Über eine nachfolgende hydrierende Aminierung des Oxoproduktes gelangt man zu Polybutyl- oder Polyisobutylaminen, die begehrte Komponenten zur Formulierung von Schmier- und Kraftstoffadditiven sind.

Bedingt durch die hohe Viskosität und die oberflächenaktiven Eigenschaften der Oxoprodukte der Polyisobutene ist aber eine wirksame Entfernung des eingesetzten Kobaltkatalysators nur schwer zu erreichen. Polyisobuten-Oxoprodukte neigen dazu, mit dem Kobaltcarbonyl eine innige Verbindung einzugehen, aus der der Katalysator kaum zu isolieren ist. Eine thermische Entkobaltung würde zwar die Carbonyle wirkungsvoll zerstören, eine Abtrennung des Kobalthydroxides durch z.B. Filtration ist aber aufgrund der hohen Viskosität nur schwer vorstellbar.

Ebenso stoßen Entkobaltungsverfahren, die auf einer Extraktion des Kobaltcarbonylwasserstoffes beruhen, sei es unter Oxidation des Kobaltes von -1 nach +2, sei es unter Beibehaltung der Oxidationsstufe, an ihre Grenzen. Hier macht sich die Neigung der Polyisobuten-Oxoprodukte nachteilig bemerkbar, mit wäßrigen Phasen stabile Emulsionen zu bilden.

Da die Hydroformylierungsreaktion mit einem Kobaltkatalysator bei erhöhten Drucken von 100 bis 400 bar durchgeführt wird, ist es in allen Verfahrensvarianten notwendig, den Rohaustrag zur weiteren Aufarbeitung auf Drucke von üblicherweise unter 50 bar zu entspannen.

Dabei kommt es insbesondere bei einer mehrphasigen Entspannung unter Einleitung einer wäßrigen Lösung für die Kobaltextraktion zu starken Scherfeldern, die die Ausbildung von Emulsionen begünstigen. Der hohe Energieeintrag bei der Dispergierung bewirkt die Bildung sehr kleiner Tröpfchen; es bildet sich eine Feindispersion oder Emulsion.

Liegen aufgrund des Phasenverhältnisses zwischen wäßriger und organischer Phase Emulsionen vom Typ einer Wasser-in-Öl-Emulsion vor, so werden diese durch das hohe Viskositätsverhältnis zwischen wäßriger und organischer Phase zusätzlich stabilisiert. Je nach Betriebsbedingungen können diese Emulsionen zwischen wäßriger und organischer Phase über mehrere Tage beständig sein. Dies hat zur Folge, daß eine Rückführung der wäßrigen Phase ebenso unmöglich ist, wie die Weiterverarbeitung des hydroformylierten Polyisobutens.

Für eine wirtschaftliche Durchführung der Hydroformylierung ist es aber erforderlich, nach dem Durchgang durch die Reaktionsstufe eine vollständige Abtrennung und Rezirkulierung des eingesetzten Kobaltkatalysators zu erreichen, da hierdurch einerseits die Kosten für das Verfahren gesenkt werden und andererseits die Weiterverarbeitung in den folgenden Verfahrensstufen wesentlich erleichtert wird.

Die Erfindung lag deshalb die Aufgabe zugrunde, die Hydroformylierung in einer Art und Weise durchzuführen, daß der eingesetzte Kobaltkatalysator möglichst vollständig abgetrennt und weitgehend im Kreis geführt werden kann, und ein für die Weiterverarbeitung geeignetes, d.h. weitgehend kobaltfreies Oxoprodukt erhalten wird.

Diese Aufgabe wurde gelöst mit einem Verfahren zur Hydroformylierung von Olefinen mit 12 bis 100 C-Atomen in Gegenwart eines Kobaltcarbonyl-Katalysators bei Drücken von 100 bis 400bar und bei einer Temperatur von 100 bis 200°C, Entspannen und Rückgewinnen des Kobaltkatalysators durch Extraktion mit einer wäßrigen sauren Lösung in Gegenwart von Luftsauerstoff, indem man die Extraktion
(a) in Gegenwart eines polymeren Emulsionsspalters, ausgewählt aus der Gruppe bestehend aus alkoxylierten Amino-, Imino- oder OH-Gruppen enthaltenden Verbindungen durchführt und
(b) zur vollständigen Phasentrennung in der noch geringe Mengen wäßrige Phase enthaltenden organischen Phase in einer Koaleszenzstufe vorzugsweise mit Hilfe einer Füllkörperkolonne, die Bildung größerer Tropfen der dispersen wäßrigen Phase bewirkt.

Als Emulsionsspalter kommen alkoxylierte Verbindungen in Betracht, wie sie üblicherweise in der Erdölindustrie zur Abtrennung des salzhaltigen Wassers Verwendung finden. Dies sind z.B. (a) mit Propylenoxid und gegebenenfalls zusätzlich Ethylenoxid alkoxylierte Oligo- und Polyamine und -imine sowie (b) alkoxylierte Alkylphenolformaldehydharze und (c) Ethylenoxid-/Propylenoxid-Blockpolymere sowie (d) deren polymere Acrylsäureester wie sie in DE-A 22 27 546 und DE-A 24 35 713 (a); DE-A 20 13 820 (b); DE-A 15 45 250 (c); und DE-A 43 26 772 (d) beschrieben sind.

Besonders bevorzugt ist die Verwendung eines Emulsionsspalters, der durch Umsetzung von Polyethylenimin mit einem MG von 10000 bis 50000 mit solchen Mengen Propylenoxid und gegebenenfalls zusätzlich Ethylenoxid erhalten wird, daß der Gehalt an Alkoxy-Einheiten 90 bis 99 Gew.-% beträgt.

Die Aufwandmenge an Demulgator, die zur Erzielung des gewünschten Effektes zudosiert wird, beträgt etwa 0,1 bis 100 g pro t des eingesetzten organischen Materials, bevorzugt 2 bis 20 g/t.

Bevorzugt wird der Demulgator in verdünnter Form kontinuierlich zugegeben. Die Verdünnung mit einem inerten Lösungsmittel, z.B. o-Xylol, erleichtert die Handhabung sowie die Dosierung der geringen benötigten Menge. Die Zugabe erfolgt zweckmäßigerweise nach der Entkobaltung, vorzugsweise zusammen mit-der Zugabe der nach der Entkobaltung, vorzugsweise zusammen mit der Zugabe der wäßrigen Extraktionslösung und der Luft bei der Entspannung, wodurch der Demulgator wirkungsvoll eingemischt wird.

Die vorbehandelte Dispersion aus wäßriger und organischer Phase kann anschließend in einer Beruhigungszone aufgetrennt werden. Dies geschieht zweckmäßigerweise in einem liegenden kontinuierlich betriebenen Phasentrenngefäß, das mit geringer Strömungsgeschwindigkeit durchflossen wird. Bedingt durch die Dichteunterschiede der Phasen trennt sich die Dispersion im Erdschwerefeld, so daß beide Phasen in geschlossener Form und weitgehend fremdphasenfrei übereinander geschichtet vorliegen. Man erhält die wäßrige Phase vollständig frei von organischer Phase, so daß die Kobaltsalzlösung ohne weitere Aufarbeitung in die Entkobaltungsstufe zurückgeführt werden kann.

Die organische Phase enthält in der Regel noch 1 bis 5 Gew.-% Fremdphasenanteil, vorzugsweise in Form feinster Tröpfchen. Obgleich der Wassergehalt selbst die Weiterverarbeitung nicht stört, sind die darin gelösten Kobaltsalze und Säuren in den nachfolgenden Umsetzungen unerwünscht.

Die Abtrennung der Restwassermenge läßt sich nun dadurch erreichen, daß man die Feinstdispersion durch eine Füllkörperschüttung leitet, vorzugsweise von oben nach unten. Durch Benetzung der großen Füllkörperoberfläche kommt es zur Oberflächenkoaleszenz und gleichzeitig durch Tropfenbewegung zur Tropfen-Tropfen-Koaleszenz. Bevorzugt werden Füllkörperkolonnen verwendet, die mit Metallringen gefüllt sind. Die sich bildenden großen Tropfen der wäßrigen Phase scheiden sich rasch ab und können als Unterphase abgezogen werden. Das Oxoprodukt wird oberhalb der Phasentrennschicht abgenommen und enthält unter üblichen Bedingungen dann noch 0,05 bis 0,3 Gew.-% suspendiertes Wasser.

Die Abtrennung der feindispergierten Restwassermenge von der organischen Phase durch Koaleszenz der Wassertröpfchen zu größeren Tropfen kann auch mit Hilfe anderer Vorrichtungen erzielt werden, z.B. mittels elektrostatischer Koalesziervorrichtungen wie sie z.B. in Chem. Ing. Techn. 62, 525 (1990) beschrieben sind, die Koaleszierung der wäßrigen Phase der Feindispersion durch Durchleiten durch eine Füllkörperschüttung, insbesondere durch eine Füllkörperkolonne, erweist sich jedoch für die Lösung der der Erfindung zugrundeliegenden Aufgabenstellung als besonders vorteilhaft und wirtschaftlich.

Die Abscheidung der Tröpfchen wird erleichtert, wenn der Apparat bei erhöhter Temperatur bei 50 bis 100°, bevorzugt 80°C betrieben wird. Die Viskosität der organischen Phase ist dann niedriger und die Sinkgeschwindigkeit der Tropfen entsprechend erhöht. Die Abscheidung der Tröpfchen kann auch bei niedrigeren Temperaturen, z.B. bei Raumtemperatur, durchgeführt werden, wobei die Abscheidungsgeschwindigkeit der Tröpfchen in Abhängigkeit von der Viskosität der organischen Phase verlangsamt ist.

Zur Entfernung von Restmengen an dispers mitgeführter kobalthaltiger, wäßriger Phase ist es zweckmäßig, eine Phasentrenneinrichtung mit handelsüblichen Koaleszierfilterkerzen nachzuschalten, in welchen die wäßrige Phase bis auf Werte entsprechend der Löslichkeit abgeschieden wird, so daß sich der Kobaltgehalt im Oxoprodukt auf Werte unter 10 ppm Co beim Polyisobuten-Oxoprodukt bzw. unterhalb der Nachweisgrenze beim C₁₃-C₂₁-Oxoprodukt, reduziert.

Die in den vorangehenden Schritten abgetrennten wäßrigen Phasen werden zweckmäßig vereinigt und in die Entkobaltung zurückgeführt. Aus diesem Kreislauf kann die zur Beschickung des Reaktors benötigte Menge an wäßriger Kobaltsalzlösung ohne besondere Maßnahmen abgezweigt werden.

Insbesondere können eventuell noch vorhandene Spuren des Emulsionsspalters in der Kobaltsalzlösung verbleiben, ohne daß es in der Hydroformylierungsreaktion zu einer unerwünschten Nebenproduktbildung kommt.

Als zu hydroformylierende Olefine kommen einerseits Polybutene und insbesondere Polyisobutene mit 28 bis 100 C-Atomen in Betracht. Insbesondere kommen Polyisobutene mit bevorzugt endständigen Doppelbindungen in Betracht, wie sie z.B. in US-A 5,286,823 beschrieben sind.

Ferner eignet sich das erfindungsgemäße Verfahren zur Hydroformylierung von C₁₂₋ bis C₂₀-Olefinen zwecks der Herstellung von Waschmittelalkoholen.

Eine vorteilhafte Ausführungsform des Verfahrens wird anhand der schematischen Zeichnung im einzelnen erläutert:

Über die Leitungen 1 bis 3 werden dem Hydroformylierungsreaktor Olefin und Synthesegas (Oxogas) sowie eine wäßrige Kobaltsalzlösung 3 zugeführt. Im Reaktor 4 findet bei üblichen Hydroformylierungsbedingungen die Umsetzung mit Oxogas zu den sauerstoffhaltigen Verbindungen statt. Der den aktiven Katalysator in Form des Kobaltcarbonylwasserstoffes enthaltende Austrag wird über Leitung 5 der Entkobaltung 6 zugeführt und dort mit Luft über Leitung 7 und einer wäßrigen sauren Kobaltsalzlösung behandelt. Dabei wechselt das Kobalt die Oxidationsstufe von -1 nach +2 und wird in der sauren wäßrigen Phase als Kobaltsalz gelöst. Unmittelbar nach der Entkobaltung, d.h. praktisch in der gleichen Stufe, wird der Demulgator über Leitung 9 zugegeben und der rohe Reaktionsaustrag über Leitung 10 in ein Phasentrenngefäß 11 geleitet. Hier trennen sich die Gasphase und die beiden Flüssigphasen. Über Leitung 12 werden die nicht umgesetzten Anteile der Luft sowie aus der Synthesestufe mitgeführte Anteile an CO und H₂ abgeleitet. Die abgeschiedene wäßrige Phase 8 wird zum Teil in die Entkobaltungsstufe 6 und zum Teil in die Hydroformylierung 4 zurückgeführt.

Als Kobaltsalzlösung für die Oxoreaktion und für die Extraktion wird bevorzugt eine Kobaltformiat- oder Kobaltacetatlösung eingesetzt, die mit Ameisensäure oder Essigsäure auf einen pH-Wert von etwa 2-5, vorteilhaft aber 3-4 eingestellt wurde. Die Konzentration des Co⁺² in der Lösung beträgt 0,5 bis 2 Gew.-%, vorteilhafterweise 1,0 bis 1,5 Gew.-%.

Die Umsetzung des Olefins im Synthesereaktor erfolgt bei 100 bis 400 bar, vorzugsweise 250 bis 300 bar, und einer Temperatur von 100 bis 200°C, bevorzugt 150 bis 190°C.

Zur Entfernung des Kobaltcarbonylwasserstoffes wird der Oxoaustrag mit molekularem Sauerstoff über Leitung 7 in Gegenwart der wäßrigsauren Kobaltlösung aus Leitung 8 behandelt. Im allgemeinen verwendet man die 0,1 bis 10-fache Menge, und besonders vorteilhaft, die 0,5 bis 0,9-fache Gewichtsmenge an wäßriger Phase, bezogen auf die organische Phase.

Der molekulare Sauerstoff wird zweckmäßig in Form von Luft eingebracht, deren Menge so bemessen ist, daß pro Grammatom Kobalt die 2 bis 2,5-fache molare Menge Sauerstoff vorhanden ist.

Die Vermischung der flüssigen Phasen und der Sauerstoff enthaltenden Gasphase kann in einem beliebigen Apparat zur Durchführung von Gas/Flüssigreaktionen erfolgen, z.B. in einer Blasensäule, einem intensiv gerührten Mischkessel oder einer Zweistoffdüse.

Nach einer bevorzugten Ausführungsform kann nach dem Passieren der Entkobaltungszone das Gemisch der drei Phasen durch eine Dispergiereinrichtung geleitet werden. Als Mischelemente dafür eignen sich übliche statische Mischer sowie Füllkörper (z.B. Raschig-Ringe, Pallringe, Glaskugeln usw.), Dadurch erreicht man eine innige Durchmischung sowie eine enge Tropfengrößenverteilung der dispersen wäßrigen Phase, was die nachfolgende Phasentrennung erleichtert.

Nach der Phasentrennung 11 wird die geringe Mengen der wäßrigen Phase enthaltende organische Phase über Leitung 13 in eine Koaleszenzstufe 14, vorzugsweise eine Füllkörperkolonne, die mit Metallfüllkörpern, z.B. Metallringen, gefüllt ist, geleitet.

Dabei wird die Füllkörperkolonne in der Regel von oben nach unten durchströmt und es werden zweckmäßigerweise Leerrohrgeschwindigkeiten von 0,1 bis 1,0 cm/sec und Verweilzeiten von 10 bis 60 Minuten eingehalten. Die Wahl der Füllkörper in Form und Material ist beliebig; sie sollten jedoch von der flüssigen Phase benetzbar sein. Dies ist im allgemeinen bei Metallfüllkörpern z.B. Ringen aus Edelstahl der Fall.

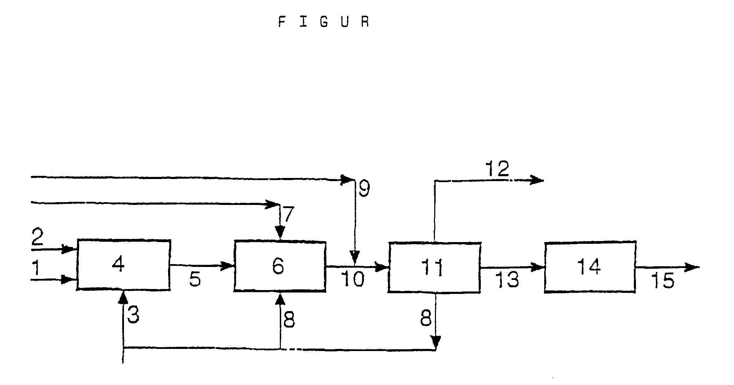

Der Koaleszenzstufe ist ein weiteres Phasentrenngefäß (in der Zeichnung nicht eingezeichnet) nachgeschaltet, in dem die in der Koaleszenzstufe vergrößerten Tröpfchen der wäßrigen Phase abgeschieden werden und man ein von wäßrigen Anteilen praktisch freies Produkt 15 erhält, das in üblicher Weise aufgearbeitet wird.

Das erfindungsgemäße Verfahren wird durch die nachfolgenden Beispiele näher erläutert.

### Beispiele

### Beispiel 1 (Vergleich; nicht erfindungsgemäß)

Es wurden stündlich 3 660 kg einer Mischung aus 1 940 kg Polyisobutenen und 1 720 kg einer C₁₂₋₁₄-Paraffinkohlenswasserstofffraktion in einen Hydroformylierungsreaktor geleitet. Gleichzeitig führte man dem Reaktor 300 kg einer wäßrigen sauren Kobaltformiatlösung zu, deren pH-Wert mit Ameisensäure auf ca. 3,4 eingestellt wurde und die 1,3 Gew-% Kobalt enthielt. Im Reaktor fand bei 180 bis 185°C die Formierung des aktiven Katalysators und eine Hydroformylierungsreaktion statt. Dem Reaktor wurde zur Aufrechterhaltung eines Gesamtdruckes von ca. 270 bar ein Gasgemisch von 40 Vol-% CO und 59 Vol-% H₂ (+1 % Inerte) so zugeführt, daß der Druck konstant blieb.

Nach dem Passieren der Reaktionsstrecke wurde das Produkt in eine Entkobaltungsstufe entspannt. Der Druck wurde dabei von 270 auf ca. 20 bar abgesenkt. In die Entkobaltungsstufe wurden pro Stunde außerdem 2 600 kg Kobaltsalzlösung der obengenannten Zusammensetzung sowie 17 kg Luft geleitet.

Nach der Entkobaltungsstufe wurden in einem Phasenschneider pro Stunde 200 kg Entspannungsgas abgetrennt und in ein Sammelsystem abgeleitet.

Die verbleibenden Flüssigphasen wurden voneinander getrennt. Die wäßrige Phase war weitgehend frei von organischen Anteilen, enthielt aber neben Co⁺² etwa 0,3 Gew.-% Kobaltcarbonyle.

Die organische Phase enthielt noch etwa 1,6 Gew.-% Fremdphase, der Gehalt an Kobalt betrug 200 ppm. Das eingesetzte Polyisobutylen wurde zu 93 % umgesetzt. 62 % des umgesetzten Polyisobutens wurden in die Wertprodukte Polyisobutylaldehyd, -alkohol oder -ester umgewandelt.

### Beispiel 2 (erfindungsgemäß)

Es wurden stündlich 3 660 kg einer Mischung aus 1 940 kg Polyisobutenen und 1 720 kg einer C₁₂₋₁₄-Paraffinkohlenwasserstofffraktion in einen Hydroformylierungsreaktor geleitet. Gleichzeitig führte man dem Reaktor 300 kg wäßrige saure Kobaltformiatlösung zu, deren pH-Wert mit Ameisensäure auf ca. 3,4 eingestellt wurde und die 1,30 Gew.% Kobalt enthielt.

Im Reaktor fand bei 180 bis 185°C die Hydroformylierungsreaktion statt. Der Reaktordruck von ca. 270 bar wurde durch Zufuhr der notwendigen Menge Oxogas konstant gehalten.

Nach dem Passieren der Reaktorstrecke wurde das Produkt in eine Entkobaltungsstufe entspannt. Der Druck wurde dabei von ca. 270 auf 20 bar abgesenkt. In die Entkobaltungszone wurden pro Stunde außerdem 2 600 kg Kobaltsalzlösung der obengenannten Zusammensetzung sowie 17 kg Luft geleitet. Unmittelbar nach dem Ausgang der Entmetallierungsstufe wurde ein Emulsionsspalter als verdünnte Lösung so zugegeben, daß.die Konzentration an Spalter 12 g pro t Reaktionsaustrag betrug. Der Emulsionsspalter war ein mit Propylenoxid modifiziertes Polyethylenimin (Molekulargew. des zur Herstellung verwendeten Polyethylenimins: ca. 20 000; Gehalt an Propoxy-Einheiten 99 Gew.-%).

Die vereinigten Ströme aus der Entkobaltungsstufe wurden durch eine mit Füllkörpern gefüllte Rohrstrecke geleitet, die Verweilzeit in der Strecke betrug ca. 1 Minute.

Nach der Mischstrecke wurden in einer Beruhigungszone pro Stunde 200 kg Entspannungsgas abgetrennt und in ein Sammelsystem abgeleitet.

Die Flüssigphasen wurden voneinander getrennt. Die wäßrige Phase war weitgehend frei von organischen Anteilen, der Gehalt an Kobaltcarbonylen betrug nur 0,05 Gew.-%.

Die organische Phase enthielt noch etwa 0,7 Gew.-% Fremdphase. Umsatz und Ausbeute entsprachen den Werten in Beispiel 1.

Zur Entfernung der Restmenge an Kobaltsalzlösung wurde der Reaktionsaustrag auf 80°C abgekühlt und durch eine Füllkörperschüttung geleitet, deren Länge 6,5 m bei 70 cm Durchmesser betrug. Die Leerrohrgeschwindigkeit in dieser Strecke lag bei etwa 0,3 cm/s. Die Strecke war senkrecht angeordnet und wurde von oben nach unten durchströmt. Die abgeschiedene wäßrige Lösung sammelte sich im unteren Teil des Apparates und wurde dort abgezogen. Die organische Phase wurde oberhalb der Phasengrenzschicht abgenommen und enthielt noch 0,14 Gew.-% Wasser. Der Kobaltgehalt der organischen Phase lag unter 10 ppm.

### Beispiel 3

Wurde als Emulsionsspalter eine Verbindung verwendet, die durch Alkoxylieren von Trimethylolpropan mit 60 Mol Propylenoxid und Mol Ethylenoxid und Veresterung des erhaltenen Polyols mit Acrylsäure hergestellt worden war, wurden ähnliche Ergebnisse mit einem Kobaltgehalt von weniger als 10 ppm erhalten.

## Patentansprüche

1. Verfahren zur Hydroformylierung von Olefinen mit 12 bis 100 C-Atomen in Gegenwart eines Kobaltcarbonyl-Katalysators bei Drücken von 100 bis 400 bar und bei einer Temperatur von 100 bis 200°C, Entspannen und Rückgewinnen des Kobaltkatalysators durch Extraktion mit einer wäßrigen sauren Lösung in Gegenwart von Luftsauerstoff, dadurch gekennzeichnet, daß man die Extraktion
(a) in Gegenwart eines polymeren Emulsionsspalters, ausgewählt aus der Gruppe bestehend aus alkoxylierten Amino-, Imino- oder OH-Gruppen enthaltenden Verbindungen durchführt und
(b) zur vollständigen Phasentrennung in der noch geringe Mengen wäßrige Phase enthaltenden organischen Phase in einer Koaleszenzstufe die Bildung größerer Tropfen der dispersen wäßrigen Phase bewirkt.

2. Verfahren gemäß Anspruch 1, dadurch gekennzeichnet, daß man als Emulsionsspalter ein mit Propylenoxid und/oder Ethylenoxid alkoxyliertes Polyethylenimin verwendet.

3. Verfahren gemäß Anspruch 1, dadurch gekennzeichnet, daß die Koaleszenzstufe aus einer Füllkörperkolonne mit nachgeschalteter Phasentrenneinrichtung besteht.

4. Verfahren gemäß Anspruch 1, dadurch gekennzeichnet, daß man in der Koaleszenzstufe (b) eine vertikal angeordnete Füllkörperkolonne verwendet, wobei die Füllkörper aus einem Material bestehen, das durch die disperse, wäßrige Phase benetzt wird und die Füllkörperschüttung durch die organische Phase geflutet ist.

5. Verfahren gemäß Anspruch 1, dadurch gekennzeichnet, daß man in der Koaleszenzstufe (b) eine mit Metallringen befüllte Füllkörperkolonne verwendet.

6. Verfahren gemäß Anspruch 1, dadurch gekennzeichnet, daß man in der Koaleszenzstufe (b) eine elektrostatische Koalesziervorrichtung verwendet.

7. Verfahren gemäß Anspruch 1, dadurch gekennzeichnet, daß man den Emulsionsspalter in Konzentrationen von 0,1 bis 100 ppm in der Extraktionsmischung anwendet.

8. Verfahren gemäß Anspruch 1, dadurch gekennzeichnet, daß man in der Koaleszenzstufe eine Temperatur zwischen 50 und 120°C einhält.

9. Verfahren gemäß Anspruch 1, dadurch gekennzeichnet, daß man die Trennung der in Stufe (b) gebildeten großen Tropfen der wäßrigen kobalthaltigen Phase und der organischen Phase mittels eines Phasentrenngefäßes unterhalb der Füllkörperschüttung vornimmt.

10. Verfahren gemäß Anspruch 1, dadurch gekennzeichnet, daß man Polyisobuten mit 28 bis 100 C-Atomen hydroformyliert.

11. Verfahren gemäß Anspruch 1, dadurch gekennzeichnet, daß man Olefine mit 12 bis 20 C-Atomen hydroformyliert.

## Claims

1. A process for the hydroformylation of olefins having from 12 to 100 carbon atoms in the presence of a cobalt carbonyl catalyst at pressures of from 100 to 400 bar and at from 100 to 200°C, depressurization and recovery of the cobalt catalyst by extraction with an aqueous acid solution in the presence of atmospheric oxygen, wherein
(a) the extraction is carried out in the presence of a polymeric emulsion breaker selected from the group consisting of alkoxylated compounds containing amino, imino or OH groups, and
(b) to achieve complete phase separation in the organic phase still containing small amounts of aqueous phase, the formation of relatively large droplets of the dispersed aqueous phase is effected in a coalescence stage.

2. A process as claimed in claim 1, wherein the emulsion breaker used is a polyethyleneimine alkoxylated with propylene oxide and/or ethylene oxide.

3. A process as claimed in claim 1, wherein the coalescence stage comprises a packed column with a downstream phase-separation apparatus.

4. A process as claimed in claim 1, wherein a vertical packed column is used in the coalescence stage (b), with the packing elements comprising a material which is wetted by the dispersed aqueous phase and the bed of packing being flooded by the organic phase.

5. A process as claimed in claim 1, wherein a packed column filled with metal rings is used in the coalescence stage (b).

6. A process as claimed in claim 1, wherein an electrostatic coalescence apparatus is used in the coalescence stage (b).

7. A process as claimed in claim 1, wherein the emulsion breaker is used in concentrations of from 0.1 to 100 ppm in the extraction mixture.

8. A process as claimed in claim 1, wherein a temperature of from 50 to 120°C is maintained in the coalescence stage.

9. A process as claimed in claim 1, wherein the separation of the large droplets of aqueous cobalt-containing phase formed in stage (b) and the organic phase is carried out by means of a phase-separation vessel below the bed of packing.

10. A process as claimed in claim 1, wherein polyisobutene having from 28 to 100 carbon atoms is hydroformylated.

11. A process as claimed in claim 1, wherein olefins having from 12 to 20 carbon atoms are hydroformylated.

## Revendications

1. Procédé d'hydroformylation d'oléfines ayant 12 à 100 atomes de carbone, en présence d'un catalyseur au carbonyl-cobalt, sous des pression de 100 à 400 bar et à une température de 100 à 200°C, détente et récupération du catalyseur au cobalt par extraction avec une solution aqueuse acide, en présence d'oxygène de l'air, caractérisé en ce que l'on met en oeuvre l'extraction
(a) en présence d'un séparateur d'émulsion polymère, choisi dans le groupe formé des composés alcoxylés contenant des groupements amino, imino ou OH, et
(b) pour une séparation complète des phases, on réalise dans une étape de coalescence dans la phase organique contenant encore de petites quantités de phase aqueuse, la formation de gouttes plus grosses de la phase aqueuse dispersée.

2. Procédé selon la revendication 1, caractérisé en ce que l'on utilise en tant que séparateur d'émulsion, une polyéthylène-imine alcoxylée avec de l'oxyde de propylène et/ou de l'oxyde d'éthylène.

3. Procédé selon la revendication 1, caractérisé en ce que l'étape de coalescence est constituée d'une colonne garnie suivie d'un dispositif de séparation de phases.

4. Procédé selon la revendication 1, caractérisé en ce que l'on utilise dans l'étape de coalescence (b) une colonne garnie disposée de façon verticale, où le garnissage est constitué d'une matière qui est mouillée par la phase aqueuse dispersée et l'amas de garnissage est noyé dans la phase organique.

5. Procédé selon la revendication 1, caractérisé en ce que l'on utilise dans l'étape de coalescence (b) une colonne garnie remplie d'anneaux métalliques.

6. Procédé selon la revendication 1, caractérisé en ce que l'on utilise dans l'étape de coalescence (b) une installation de coalescence électrostatique.

7. Procédé selon la revendication 1, caractérisé en ce que l'on utilise le séparateur d'émulsion à des concentrations de 0,1 à 100 ppm dans le mélange d'extraction.

8. Procédé selon la revendication 1, caractérisé en ce que l'on maintient dans l'étape de coalescence une température entre 50 et 120°C.

9. Procédé selon la revendication 1, caractérisé en ce que l'on effectue la séparation des grosses gouttes de phase aqueuse contenant du cobalt, formées dans l'étape b), et de la phase organique au moyen d'un récipient de séparation de phases sous l'amas de garnissage.

10. Procédé selon la revendication 1, caractérisé en ce que l'on hydroformyle du polyisobutène ayant 28 à 100 atomes de carbone.

11. Procédé selon la revendication 1, caractérisé en ce que l'on hydroformyle des oléfines ayant 12 à 20 atomes de carbone.
